# FASCICULE DE BREVET EUROPEEN

(11) **EP 4 466 068 B1**
(45) Date de publication et mention de la délivrance du brevet: **01.04.2026**
(21) Numéro de dépôt: 23705621.3
(22) Date de dépôt: 19.01.2023
(51) Int. Cl.: A61N 5/06, A61B 18/18

(54) **APPAREIL A MAIN A LUMIERE INTENSE PULSEE A GUIDE DE LUMIERE**
TRAGBARE LICHTLEITERHALTIGE VORRICHTUNG ZUR EMISSION VON INTENSIVEM GEPULSTEM LICHT
HAND-HELD LIGHT-GUIDE-COMPRISING APPARATUS FOR EMITTING INTENSE PULSED LIGHT

(30) Priorité: 19.01.2022 FR 2200450
(43) Date de publication de la demande: 27.11.2024
(73) Titulaire: Quantel Medical, 63800 Cournon-d'Auvergne (FR)
(72) Inventeur: WASSMER, Benjamin, 63000 CLERMONT FERRAND (FR); SECOND, Thomas, 77310 SAINT-FARGEAU PONTHIERRY (FR); PUREUR, David, 63000 CLERMONT FERRAND (FR)
(74) Mandataire: Regimbeau
(86) Numéro de dépôt international: PCT/FR2023/050074
(87) Numéro de publication internationale: WO 2023/139334

(56) Documents cités:
- WO-A1-2017/223331
- FR-A1- 2 929 832
- FR-A1- 2 934 951
- US-A- 5 683 380
- US-A1- 2002 173 780
- US-A1- 2011 213 447

## Description

### DOMAINE TECHNIQUE

La présente invention appartient au domaine des système de photo-stimulation du corps humain, et plus précisément se rapporte à un appareil à main à lumière intense pulsée à guide de lumière, en particulier pour des applications ophtalmiques.

### ETAT DE LA TECHNIQUE

Le principe de la lumière intense pulsée (ou "Intense Light Pulse", IPL, en anglais) repose sur l'émission d'une lumière vers la peau. Un système à lumière intense pulsée comprend une console et un appareil à main. L'opérateur règle les paramètres de l'impulsion de lumière (durée, intensité...) sur la console puis mets l'appareil à main en contact sur la zone du patient à traiter, et enfin déclenche le tir de lumière. La lumière interagit avec des composants de la peau ou des composants sous-cutanées, en fonction des longueurs d'onde. Le temps d'impact de l'impulsion lumineuse peut varier de 1 ms à 100 ms.

L'appareil à main à lumière intense pulsée comprend ainsi un corps configuré pour être tenu à la main, ledit corps présentant une ouverture accueillant une interface de traitement configurée pour être placé contre une peau. Il est possible d'appliquer un gel pour garantir un bon contact et une bonne transmission lumineuse.

Toutefois, les appareils à main à lumière intense pulsée actuels ne donnent pas entièrement satisfaction. Ils souffrent en effet d'un manque de précision, la configuration actuelle ne permettant pas à l'opérateur de visualiser correctement la zone traitée. De plus, l'uniformité de l'émission lumineuse sur la zone de traitement est généralement faible. Dans ce contexte, il est fait référence au document US2002/0173780 A1.

De telles limites ne sont pas problématiques lorsque l'appareil à main à lumière intense pulsée est utilisé pour des applications ne requérant pas une grande précision, comme par exemple des esthétiques de la peau, principalement l'épilation définitive ou semi-définitive (photo-épilation), l'épilation semi-définitive, ou le traitement des signes du vieillissement tels que les taches pigmentaires (photo-dépigmentation). En revanche, des applications telles que des soins peuvent nécessiter plus de précision dans la zone de traitement, et les appareils à main actuels n'y sont pas adaptés. C'est par exemple le cas pour des applications ophtalmiques telles qu'une stimulation lumineuse des canaux lacrymaux, où la zone traitée doit être précisément localisée sous l'œil. Si celle-ci est trop éloignée de l'œil, la stimulation ne provoquera pas l'effet escompté, alors qu'à l'inverse être trop proche de l'œil peut être dangereux. Il devient alors d'autant plus important que l'ensemble de la zone traitée reçoive une quantité de lumière adéquate, puisqu'il ne peut être question de déplacer l'appareil à main pour lisser les inhomogénéités.

### PRESENTATION DE L'INVENTION

L'invention a pour but de proposer un appareil à main permettant une précision spatiale accrue pour illuminer de façon homogène une zone de la peau à traiter.

A cet effet, il est proposé un appareil à main à lumière intense pulsée comprenant un corps configuré pour être tenu à la main, ledit corps présentant une ouverture accueillant une interface de traitement configurée pour être placé contre une peau, le corps logeant une lampe adaptée pour émettre une impulsion lumineuse à travers l'interface de traitement dans une direction d'émission, dans lequel l'interface de traitement comprend un guide de lumière faisant saillie du corps de l'appareil à main, le guide de lumière formant un polyèdre ayant des faces extrémales opposées dans la direction d'émission et des faces latérales reliant lesdites faces extrémales, le guide de lumière présentant une épaisseur, entre les faces extrémales dans la direction d'émission, supérieure à 6 mm, et les faces latérales présentant une rugosité avec un écart moyen arithmétique Ra inférieur ou égal à 3,2 et supérieur à 0,2.

L'appareil est avantageusement complété par les caractéristiques suivantes, prises seules ou en une quelconque de leur combinaison techniquement possible :
- le guide de lumière présente une face extrémale présentant une surface s'étendant selon un axe court compris entre 15 mm et 30 mm, et un axe long compris entre 30 mm et 60 mm ;
- le guide de lumière fait saillie d'au moins 5 mm par rapport au corps ;
- les faces latérales présentent une rugosité avec un écart moyen arithmétique Ra supérieur à 0,2 ;
- un état de surface des faces latérales résulte d'une découpe du guide de lumière dans du verre ;
- l'appareil à main comprend en outre un capot opaque entourant les faces latérales ;
- le guide de lumière est formé d'un matériau présentant un indice de réfraction supérieur à 1,45 ;
- l'appareil à main comprend un filtre disposé entre la lampe et le guide de lumière, configuré pour filtrer au moins les longueurs d'onde inférieures à 580 nm, le filtre et le guide de lumière étant formés de matériaux différents ;
- le guide de lumière est formé d'un pavé en verre.

L'invention concerne également un système à lumière intense pulsée comprend un appareil à main selon l'invention, et une console à laquelle est relié ledit appareil à main.

### PRESENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :
- la figure 1 montre de manière simplifiée un instrument à main selon un mode de réalisation possible de l'invention ;
- la figure 2 montre schématiquement un ensemble d'illumination comprenant une lampe et un guide de lumière selon un mode de réalisation possible de l'invention ;
- la figure 3 montre l'ensemble de la figure 2 selon un angle différent ;
- la figure 4 montre schématiquement un ensemble d'illumination comprenant une lampe, un guide de lumière et un capot selon un mode de réalisation possible de l'invention
- la figure 5 montre l'ensemble de la figure 4 selon un angle différent ;
- la figure 6 est un graphique illustrant la répartition du flux lumineux le long d'un axe long en sortie du guide de lumière, selon plusieurs configurations de l'appareil à main ;
- la figure 7 est un graphique illustrant la répartition du flux lumineux le long d'un axe court en sortie du guide de lumière, selon plusieurs configurations de l'appareil à main.

### DESCRIPTION DETAILLEE

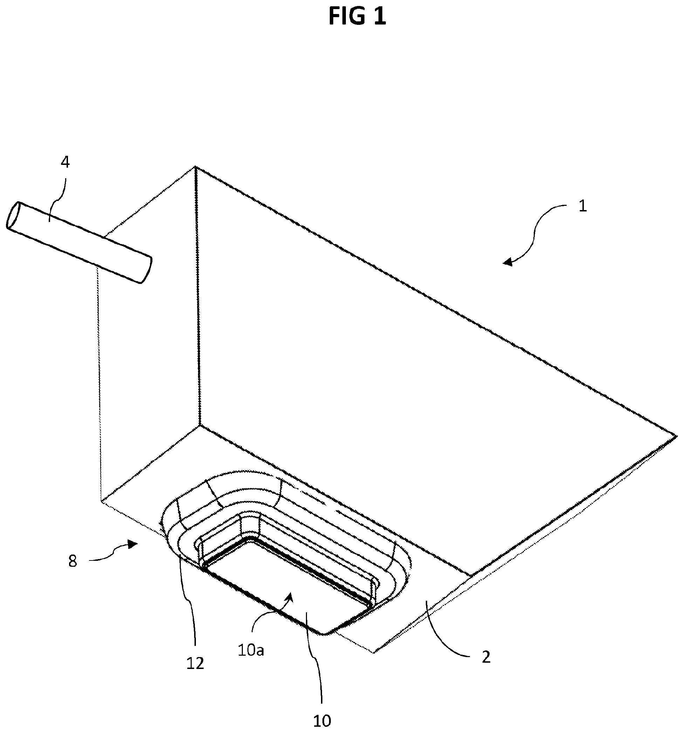

En référence à la Figure 1, l'appareil à main 1 à lumière intense pulsée comprend un corps 2 configuré pour être tenu à la main. L'appareil à main 1 est typiquement relié à une console, et comprend donc un connecteur filaire 4 permettant l'alimentation en électricité de l'appareil à main 1. La console intègre tout l'électronique nécessaire pour charger et décharger des capacités en haute tension (300 à 1000 V typiquement). Le connecteur filaire 4 peut aussi intégrer une circulation d'eau entre la console et l'appareil à main 1 à des fins de refroidissement. La console intègre également des moyens de réglage permettant de régler les paramètres de l'impulsion de lumière (durée, intensité...).

Le corps 2 présente une ouverture accueillant une interface de traitement 8 configurée pour être placé contre une peau, c'est-à-dire au contact de cette peau ou du moins très proche de celle-ci (moins de 1 cm). L'interface de traitement 8 comprend un guide de lumière 10 faisant saillie du corps 2 de l'appareil à main 1, dans la direction d'émission. De préférence, le guide de lumière 10 fait saillie d'au moins 5 mm par rapport au corps 2, et de préférence d'au moins 7 mm. La saillie du guide de lumière 10 s'entend de la hauteur à laquelle s'élève le guide de lumière 10 par rapport à la paroi du corps 2 qui entoure le guide de lumière 10 dans la direction d'émission.

Dans cet exemple, l'interface de traitement 8 comprend un capot 12 entourant le guide de lumière 10, notamment en périphérie de l'ouverture du corps 2. Le capot 12 laisse libre une face 10a du guide de lumière 10 dans une direction d'émission de la lumière, qui est la face 10a destinée à être placée contre la peau.

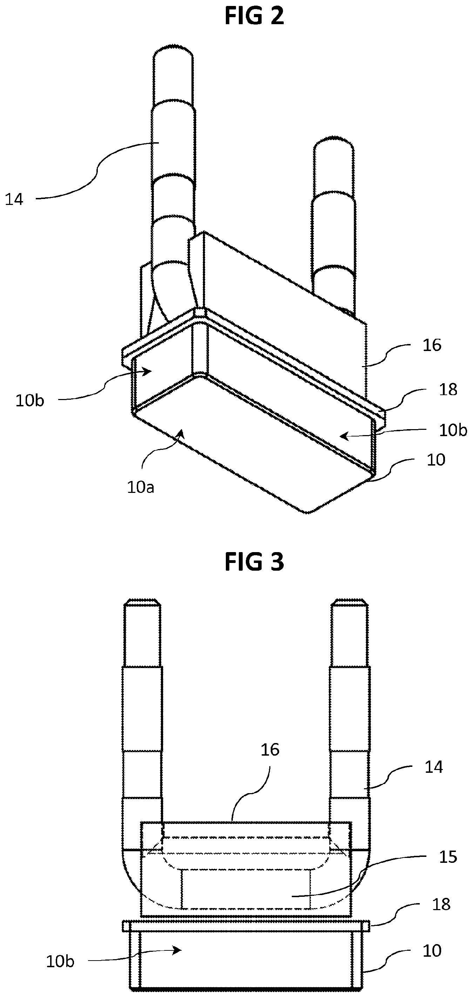

En référence à la Figure 2 et à la Figure 3 illustrant un ensemble d'illumination, le corps 2 de l'appareil à main 1 loge une lampe 14 adaptée pour émettre une impulsion lumineuse à travers l'interface de traitement 8 dans une direction d'émission. La lampe 14 est de préférence une lampe flash. Lors de l'émission d'une impulsion lumineuse, une haute tension se décharge à travers la lampe 14 en émettant une lumière de haute énergie (10 à 200 Joules) durant un temps très court (1 à 10 ms) et sur un large spectre de longueur d'onde (400 à 1200 nm). Dans l'exemple illustré, la lampe 14 prend une forme de U, la partie qui émet la lumière utile étant située dans la jonction 15 des branches de ce U, au niveau de laquelle un réflecteur 16 entoure la lampe 14 sauf dans la direction d'émission. Le réflecteur 16 permet de renvoyer vers la sortie de l'interface de traitement 8 des rayons qui autrement iraient échauffer le corps 2 de l'appareil à main 1. Typiquement le réflecteur 16 est une pièce opaque, réfléchissante ou au moins blanche afin de renvoyer la lumière, et de préférence très diffusante afin d'homogénéiser la lumière renvoyée. De préférence, l'appareil à main 1 est configuré pour accueillir une circulation d'eau au contact de la lampe 14, et de préférence entre le réflecteur 16 et la lampe 14.

L'ensemble d'illumination comprend typiquement un filtre 18 disposé en sortie de la lampe 14 dans la direction d'illumination, pour réduire le spectre de la lumière émise en fonction des applications. De préférence, un tel filtre 18 est configuré pour filtrer les longueurs d'onde de l'impulsion lumineuse inférieures à 580 nm (fréquence de coupure ou transmittance inférieure à 0,1), et de préférence inférieures à 600 nm. Par exemple, le filtre 18 est un filtre passe-Haut SCHOTT RG-610 avec une fréquence de coupure de 610 nm. Le filtrage des basses fréquences permet notamment de diminuer la sensibilité de la stimulation lumineuse à la teinte de la peau. Typiquement, le filtre 18 est plan et présente une épaisseur de 1 à 4 mm, de préférence inférieure à 3 mm.

Un guide de lumière 10 est disposé après le filtre 18 dans la direction d'émission. Le guide de lumière 10 forme un polyèdre ayant des faces extrémales 10a opposées dans la direction d'émission et des faces latérales 10b reliant lesdites faces extrémales 10a. Typiquement, le guide de lumière 10 est un prismatoïdes hexaédraux à face quadrilatères, et plus précisément un cuboïde tel que le pavé droit illustré. Le guide de lumière présente une épaisseur, dans la direction d'émission, supérieure à 6 mm, et de préférence supérieure à 8 mm, et de préférence encore supérieure à 11 mm. L'épaisseur doit être suffisante pour permettre une répartition homogène de la lumière sur la zone à traiter. Elle ne doit toutefois pas être trop longue afin de ne pas trop réduire l'énergie transmise. De préférence, l'épaisseur est inférieure à 30 mm.

A titre d'exemple, la face extrémale 10a peut présenter une surface s'étendant selon un axe court compris entre 15 mm et 30 mm, et un axe long compris entre 30 mm et 60 mm.

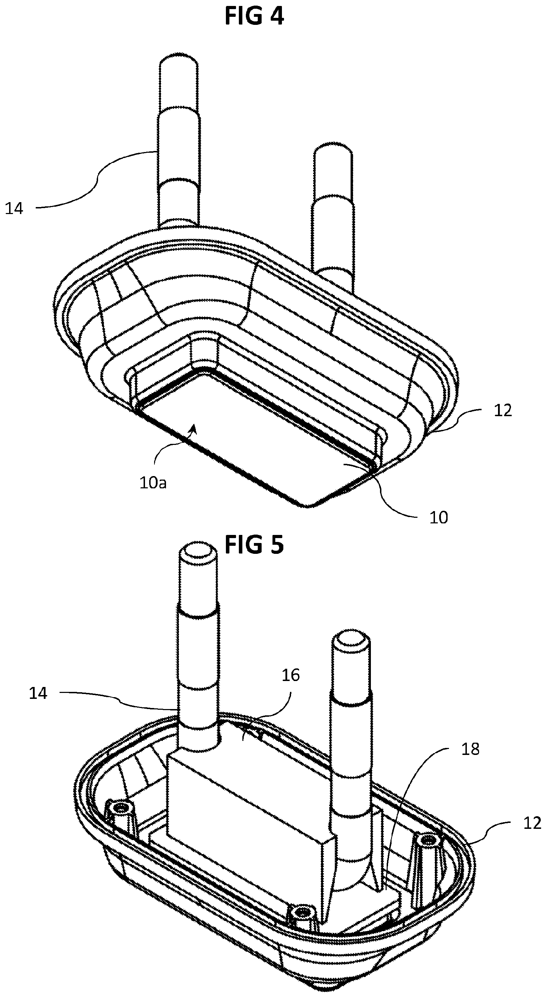

Comme illustré sur la Figure 4 et la Figure 5, le capot 12 peut recouvrir les faces latérales 10b du guide de lumière 10. Le capot 12 permet de se prémunir d'un danger sur la lumière diffusée par les faces latérales 10b du guide de lumière 10 sans modifier ni l'énergie finale transmise ni le profil transverse de la lumière transmise. De fait, le capot 12, ou du moins une partie ce celui-ci, fait saillie par rapport au corps 2 de l'appareil à main 1 dans la direction d'émission, de préférence sur la même hauteur que le guide de lumière 10. Une partie du capot 12 est logé dans le corps 2.

Le guide de lumière 10 est réalisé dans un matériau transparent, susceptible de laisser passer l'énergie d'illumination. Typiquement, le guide de lumière 10 est formé d'une seule pièce. De préférence, le guide de lumière 10 est en verre, et par exemple en verre borosilicate tel que le N-BK7 ou un verre dit flint dense tel que le SF11 de Schott. De préférence, le guide de lumière présente un haut indice optique, c'est-à-dire un indice de réfraction supérieur à 1,45, de préférence supérieur à 1,51, et de préférence supérieur à 1,60, et de préférence encore supérieur à 1,70. Avoir un verre à haute indice optique permet d'avoir une réflexion totale interne plus importante, et permet donc d'avoir un meilleur guidage de la lumière dans la direction d'émission pour réduire les pertes d'énergie et éviter d'avoir une émission lumineuse à travers les faces latérales 10b, qui pourraient être dangereuses. De préférence, le filtre 18 et le guide de lumière 10 sont formés de matériaux différents.

Afin de s'assurer non seulement d'un traitement uniforme de la zone à traiter, mais également pour qu'il puisse être assuré que l'ensemble de la zone recevant la lumière en reçoive suffisamment afin de garantir une précision de localisation du traitement, il est important de s'assurer d'avoir un profil d'émission transverse en sortie le plus homogène possible. A cet égard, l'état de surface des faces latérales 10b du guide de lumière peut être utilisé pour améliorer la constance du profil d'émission transverse.

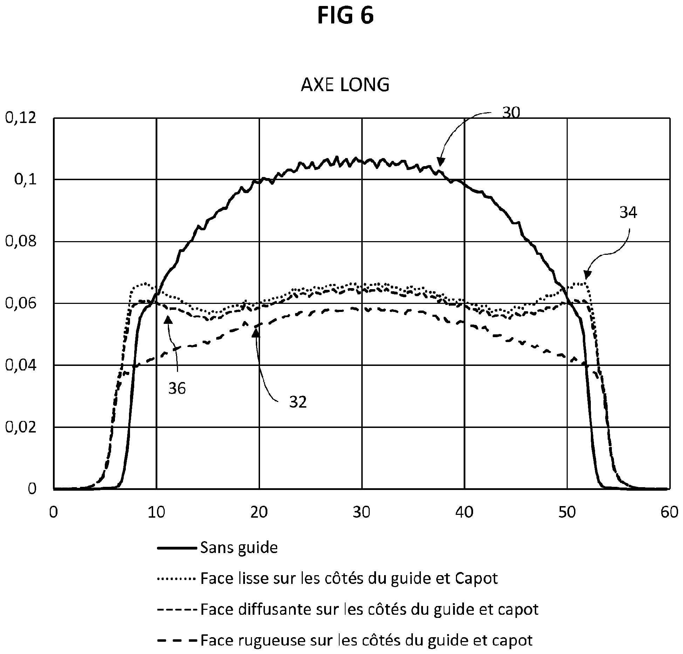

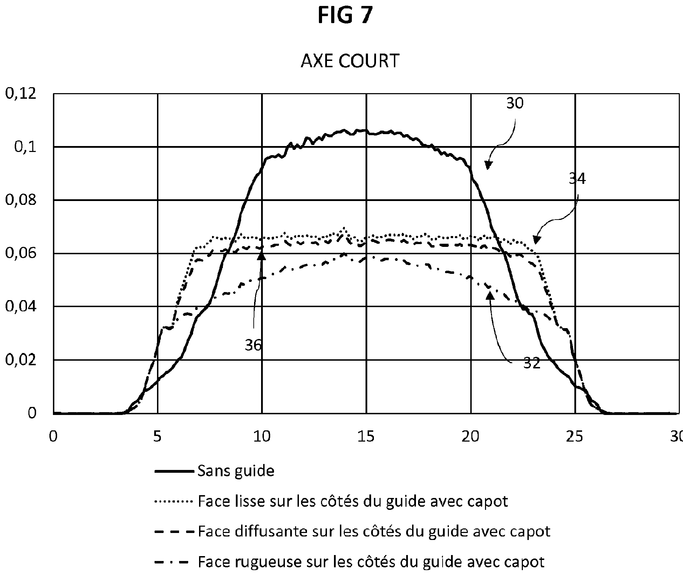

La figure 6 est montre la répartition du flux lumineux le long d'un axe long en sortie du guide de lumière, selon plusieurs configurations de l'appareil à main, tandis que la Figure 7 la montre le long d'un axe court. Les axes des abscisses sont en millimètres, tandis que l'axe des ordonnées est en mesure arbitraire représentative de l'intensité moyenne, sur un axe perpendiculaire à l'axe en abscisse, de l'énergie reçue sur une zone de réception.

La première courbe 30 en traits continus correspond au profil d'émission transverse d'un appareil à main 1 sans guide de lumière 10 ni capot 12. La deuxième courbe 32 en tirets et points correspond au profil d'émission transverse d'un appareil à main 1 avec un guide de lumière 10 et capot 12, le guide de lumière 10 présentant des faces latérales 10b rugueuses. La troisième courbe 34 en pointillés correspond au profil d'émission transverse d'un appareil à main 1 avec un guide de lumière 10 et capot 12, le guide de lumière 10 présentant des faces latérales 10b lisses. La quatrième courbe 36 en tirets correspond au profil d'émission transverse d'un appareil à main 1 avec un guide de lumière 10 et capot 12, le guide de lumière 10 présentant des faces latérales 10b diffusantes. Il est à noter que la présence ou l'absence d'un capot 12 ne changeant pas les résultats, il n'a pas été représenté de configuration avec guide de de lumière 20 mais sans capot 12. Dans toutes les configurations, les formes et matériaux du guide de lumière 10 sont les mêmes (pavé de 47 mm par 19 mm en BK7).

On constate tout d'abord qu'un appareil à main 1 sans guide de lumière 10 présente un profil d'émission transverse (première courbe 30) variant énormément avec l'abscisse, aussi bien sur l'axe long que sur l'axe court, avec une forme de cloche, ce qui n'est pas souhaitable. Un appareil à main 1 avec un guide de lumière 10 à faces latérales rugueuse présente un profil d'émission transverse (deuxième courbe 32) qui varie moins, mais qui prend des valeurs sensiblement plus basses, indiquant une perte importante d'énergie. Un appareil à main 1 avec un guide de lumière 10 à faces latérales lisses présente un profil d'émission transverse (troisième courbe 34) pratiquement plane et avec des valeurs plus élevées que celles obtenues avec des faces rugueuses. Une faible rugosité des faces latérales permet donc d'augmenter à la fois la constance du profil d'émission transverse, et la quantité d'énergie transmise, limitant ainsi les pertes.

Il apparaît cependant que les faces latérales n'ont pas besoin d'être nécessairement lisses pour que le guide de lumière 10 présente des caractéristiques satisfaisantes. Un appareil à main 1 avec un guide de lumière 10 à faces latérales diffusantes présente un profil d'émission transverse (quatrième courbe 36) avec des qualités similaires à celles obtenues avec des faces lisses, tant en planéité du profil d'émission transverse qu'en quantité d'énergie transmise.

Une face lisse est obtenue en polissant cette face après découpe du matériau du guide de lumière 10, et présente donc une rugosité avec un écart moyen arithmétique Ra inférieur ou égal à 0,2. Une face latérale diffusante est obtenue en conservant l'état de surface après découpe du matériau du guide de lumière 10, ou avec un polissage minime, et présente une rugosité avec un écart moyen arithmétique Ra inférieur ou égal à 3,2. Une face rugueuse désigne une face avec une rugosité avec un écart moyen arithmétique Ra strictement supérieur à 3,2. Une telle rugosité est par exemple obtenue au moyen d'un traitement de surface des faces latérales 10b tel qu'un dépolissage ou sablage.

Ainsi, l'utilisation d'un guide de lumière 10 avec des faces latérales présentant une rugosité avec un écart moyen arithmétique Ra inférieur ou égal à 3,2 permet d'obtenir les qualités de précision recherchées. Dans la mesure où chaque intervention implique des coûts, il est préférable d'utiliser un guide de lumière 10 en verre découpé et brute sans polissage ni traitement puisque le polissage ne permet pas d'obtenir des gains justifiant les coûts supplémentaires. Par conséquent, les faces latérales 10b présentent une rugosité avec un écart moyen arithmétique Ra supérieur à 0,2, et de préférence supérieur à 0,5. De préférence, l'état de surface des faces latérales résulte d'une découpe du guide de lumière 10 dans du verre.

L'invention n'est pas limitée au mode de réalisation décrit et représenté aux figures annexées. Des modifications restent possibles, notamment du point de vue de la constitution des divers éléments ou par substitution d'équivalents techniques, sans sortir pour autant du domaine de protection de l'invention.

## Revendications

1. Appareil à main (1) à lumière intense pulsée comprenant un corps (2) configuré pour être tenu à la main, ledit corps (2) présentant une ouverture accueillant une interface de traitement (8) configurée pour être placé contre une peau, le corps (2) logeant une lampe (14) adaptée pour émettre une impulsion lumineuse à travers l'interface de traitement (8) dans une direction d'émission,
**caractérisé en ce que** l'interface de traitement (8) comprend un guide de lumière (10) faisant saillie du corps (2) de l'appareil à main (1), le guide de lumière (10) formant un polyèdre ayant des faces extrémales (10a) opposées dans la direction d'émission et des faces latérales (10b) reliant lesdites faces extrémales, le guide de lumière (10) présentant une épaisseur, entre les faces extrémales (10a) dans la direction d'émission, supérieure à 6 mm, et les faces latérales (10b) présentant une rugosité avec un écart moyen arithmétique Ra inférieur ou égal à 3,2 et supérieur à 0,2.

2. Appareil à main selon l'une quelconque des revendications précédentes, dans lequel le guide de lumière (10) présente une face extrémale (10a) présentant une surface s'étendant selon un axe court compris entre 15 mm et 30 mm, et un axe long compris entre 30 mm et 60 mm.

3. Appareil à main selon l'une quelconque des revendications précédentes, dans lequel le guide de lumière (10) fait saillie d'au moins 5 mm par rapport au corps (2).

4. Appareil à main selon l'une quelconque des revendications précédentes, dans lequel les faces latérales (10b) présentent une rugosité avec un écart moyen arithmétique Ra supérieur à 0,5.

5. Appareil à main selon l'une quelconque des revendications précédentes, dans lequel le guide de lumière est formé dans en verre découpé et brute sans polissage ni traitement.

6. Appareil à main selon l'une quelconque des revendications précédentes, comprenant en outre un capot (12) opaque entourant les faces latérales (10b).

7. Appareil à main selon l'une quelconque des revendications précédentes, dans lequel le guide de lumière (10) est formé d'un matériau présentant un indice de réfraction supérieur à 1,45

8. Appareil à main selon l'une quelconque des revendications précédentes, comprenant un filtre (18) disposé entre la lampe et le guide de lumière (10), configuré pour filtrer au moins les longueurs d'onde inférieures à 580 nm, le filtre (18) et le guide de lumière (10) étant formés de matériaux différents.

9. Appareil à main selon l'une quelconque des revendications précédentes, dans lequel le guide de lumière (10) est formé d'un pavé en verre.

10. Système à lumière intense pulsée comprend un appareil à main selon l'une quelconque des revendications précédentes, et une console à laquelle est relié ledit appareil à main.

## Patentansprüche

1. Handgehaltenes Gerät (1) mit intensiv gepulstem Licht, umfassend ein Körper (2), der dazu konfiguriert ist, in der Hand gehalten zu werden, wobei der Körper (2) eine Öffnung aufweist, die eine Behandlungsschnittstelle (8) aufnimmt, die dazu konfiguriert ist, an die Haut angelegt zu werden, wobei der Körper (2) eine Lampe (14) unterbringt, die dazu geeignet ist, einen Lichtimpuls durch die Behandlungsschnittstelle (8) hindurch in einer Emissionsrichtung zu emittieren,
**dadurch gekennzeichnet, dass** die Behandlungsschnittstelle (8) einen Lichtleiter (10) umfasst, der von dem Körper (2) des handgehaltenen Geräts (1) aus vorsteht, wobei der Lichtleiter (10) ein Polyeder bildet, das extremale Seiten (10a), die in der Emissionsrichtung entgegengesetzt sind, und laterale Seiten (106), welche die extremalen Seiten verbinden, aufweist, wobei der Lichtleiter (10) in der Emissionsrichtung zwischen den extramalen Seiten (10a) eine Dicke aufweist, die größer als 6 mm ist, und die lateralen Seiten (10b) eine Rauheit mit einer arithmetischen mittleren Abweichung Ra, die kleiner oder gleich 3,2 und größer als 0,2 ist, aufweisen.

2. Handgehaltenes Gerät nach einem der vorhergehenden Ansprüche, wobei der Lichtleiter (10) eine extremale Seite (10a) aufweist, die eine Fläche aufweist, die sich entlang einer kurzen Achse, die zwischen 15 mm und 30 mm misst, und einer langen Achse, die zwischen 30 mm und 60 mm misst, erstreckt.

3. Handgehaltenes Gerät nach einem der vorhergehenden Ansprüche, wobei der Lichtleiter (10) im Verhältnis zu dem Körper (2) um mindestens 5 mm vorsteht.

4. Handgehaltenes Gerät nach einem der vorhergehenden Ansprüche, wobei die lateralen Seiten (10b) eine Rauheit mit einer arithmetischen mittleren Abweichung Ra von mehr als 0,5 aufweisen.

5. Handgehaltenes Gerät nach einem der vorhergehenden Ansprüche, wobei der Lichtleiter aus zugeschnittenem und rohem Glas ohne Schleifen oder Behandlung gebildet ist.

6. Handgehaltenes Gerät nach einem der vorhergehenden Ansprüche, ferner umfassend eine undurchsichtige Abdeckung (12), welche die lateralen Seiten (10b) umgibt.

7. Handgehaltenes Gerät nach einem der vorhergehenden Ansprüche, wobei der Lichtleiter (10) aus einem Material gebildet ist, das einen Brechungsindex von mehr als 1,45 aufweist.

8. Handgehaltenes Gerät nach einem der vorhergehenden Ansprüche, umfassend ein Filter (18), das zwischen der Lampe und dem Lichtleiter (10) angeordnet ist und dazu konfiguriert ist, mindestens die Wellenlängen kleiner als 580 nm zu filtern, wobei das Filter (18) und der Lichtleiter (10) aus verschiedenen Materialien gebildet sind.

9. Handgehaltenes Gerät nach einem der vorhergehenden Ansprüche, wobei der Lichtleiter (10) aus einem Glasstein gebildet ist.

10. System mit intensiv gepulstem Licht, das ein handgehaltenes Gerät nach einem der vorhergehenden Ansprüche und eine Konsole, mit der das handgehaltene Gerät verbunden ist, umfasst.

## Claims

1. A hand-held apparatus (1) for emitting intense pulsed light comprising a body (2) configured to be hand held, said body (2) having an aperture accommodating a treatment interface (8) configured to be placed against a skin, the body (2) housing a lamp (14) suitable for emitting a light pulse through the treatment interface (8) in an emission direction,
**characterized in that** the treatment interface (8) comprises a light guide (10) that protrudes from the body (2) of the hand-held apparatus (1), the light guide (10) forming a polyhedron having end faces (10a) that are opposite in the emission direction and side faces (10b) joining said end faces, the light guide (10) having a thickness, between the end faces (10a) in the emission direction, larger than 6 mm, and the side faces (10b) having an arithmetic mean roughness Ra lower than or equal to 3.2 and greater than 0.2.

2. The hand-held apparatus according to any one of the preceding claims, wherein the light guide (10) has an end face (10a) having a surface extending along a short axis comprised between 15 mm and 30 mm, and a long axis comprised between 30 mm and 60 mm.

3. The hand-held apparatus according to any one of the preceding claims, wherein the light guide (10) protrudes at least 5 mm relative to the body (2).

4. The hand-held apparatus according to any one of the preceding claims, wherein the side faces (10b) have an arithmetic mean roughness Ra greater than 0.5.

5. The hand-held apparatus according to any one of the preceding claims, wherein the light guide is formed from cut and raw glass without polishing or treatment.

6. The hand-held apparatus according to any one of the preceding claims, further comprising an opaque cover (12) surrounding the side faces (10b).

7. The hand-held apparatus according to any one of the preceding claims, wherein the light guide (10) is formed of a material having a refractive index greater than 1.45.

8. The hand-held apparatus according to any one of the preceding claims, comprising a filter (18) disposed between the lamp and the light guide (10), configured to filter at least wavelengths less than 580 nm, the filter (18) and the light guide (10) being formed of different materials.

9. The hand-held apparatus according to any one of the preceding claims, wherein the light guide (10) is formed of a glass block.

10. An intense pulsed light system comprises a hand-held apparatus according to any one of the preceding claims, and a console to which said hand-held apparatus is connected.
